# EUROPÄISCHE PATENTSCHRIFT

(11) **EP 4 255 778 B1**
(45) Veröffentlichungstag und Bekanntmachung des Hinweises auf die Patenterteilung: **19.06.2024**
(21) Anmeldenummer: 21816416.8
(22) Anmeldetag: 19.11.2021
(51) Int. Cl.: B60S 1/64, B62D 1/183, A61L 2/00

(54) **FAHRZEUG**
VEHICLE
VÉHICULE

(30) Priorität: 07.12.2020 DE 102020007463
(43) Veröffentlichungstag der Anmeldung: 11.10.2023
(73) Patentinhaber: Mercedes-Benz Group AG, 70372 Stuttgart (DE)
(72) Erfinder: SCHADT, Torsten, 71034 Böblingen (DE)
(74) Vertreter: Novagraaf Group
(86) Internationale Anmeldenummer: PCT/EP2021/082360
(87) Internationale Veröffentlichungsnummer: WO 2022/122349

(56) Entgegenhaltungen:
- CN-U- 208 897 149
- DE-A1-102013 002 539
- DE-A1-102017 211 515
- DE-A1-102019 114 717

## Beschreibung

Die vorliegende Erfindung betrifft ein Fahrzeug mit den Merkmalen des Oberbegriffs des Patentanspruchs 1.

Aus unterschiedlichen Gründen besteht bei einem Fahrzeug der Bedarf, die Greiffläche des Lenkeingabegeräts zu reinigen und insbesondere zu desinfizieren. Beispielsweise kann ein erhöhter Hygienebedarf im Hinblick auf die Ausbreitung bzw. Übertragung von Krankheiten bestehen. Ebenso kann die Nutzung von Fahrzeugen durch verschiedene Fahrer, z. B. in einem Fuhrpark oder beim Car-Sharing, den Bedarf für eine gereinigte Greiffläche am Lenkeingabegerät erhöhen.

Ein gattungsgemäßes Fahrzeug ist beispielsweise aus der DE 10 2013 002 539 A1 bekannt und ist mit einer Lenkeinrichtung zum Lenken des Fahrzeugs ausgestattet, die ein manuell betätigbares Lenkeingabegerät, z.B. in Form eines Lenkrads, zum manuellen Greifen und Eingeben von Lenkbefehlen aufweist, wobei das Lenkeingabegerät eine einteilige oder mehrteilige Greiffläche aufweist, die vom Fahrzeugführer zum Eingeben von Lenkbefehlen manuell greifbar ist. Das gattungsgemäße Fahrzeug ist außerdem mit einer Reinigungsvorrichtung zum Reinigen der Greiffläche ausgestattet. Beim bekannten Fahrzeug umfasst die Reinigungsvorrichtung ein z.B. im Armaturenbrett untergebrachtes Gehäuse, in dem ein Reinigungskopf der Reinigungsvorrichtung untergebracht ist. Für einen Reinigungsvorgang kann nun dieser Reinigungskopf aus dem Gehäuse ausgeklappt bzw. ausgefahren werden, sodass der Reinigungskopf für den Reinigungsbetrieb einen Umfangsabschnitt des als Lenkrad ausgestalteten Lenkeingabegeräts umgreift. Durch eine Drehbewegung des Lenkrads um dessen Lenkradachse wird eine Relativbewegung zwischen der Greiffläche und dem Reinigungskopf erreicht, sodass die Greiffläche über den gesamten Umfang des Lenkrads gereinigt werden kann. Der Reinigungskopf ist mit Mitteln zur Ausbringung eines Fluids auf das jeweils umgriffene Umfangssegment des Lenkrads ausgestattet,
wobei das Fluid ein Reinigungs- und/oder Desinfektionsmittel enthält. Ferner kann im Reinigungskopf eine Reinigungsfläche für eine mechanische Reinigung des aufgenommenen Umfangsabschnitts und/oder eine Strahlenquelle zur Aussendung elektromagnetischer Strahlung zur Abtötung von Keimen und Organismen vorgesehen sein. Beim bekannten Fahrzeug muss das Lenkrad, um es reinigen zu können, aus seiner, vom jeweiligen Fahrer eingestellten aktuellen Gebrauchsstellung in eine Ausgangsstellung überführt werden, in welcher der Reinigungskopf den jeweiligen Umfangsabschnitt des Lenkrads umgreifen kann. Somit ist eine vergleichsweise komplexe Synchronisation unterschiedlicher Einrichtungen erforderlich, um den Reinigungsvorgang durchzuführen.

Aus der DE 10 2019 114 717 A1 ist ein Fahrzeug bekannt, welches ein manuell betätigbares Lenkeingabegerät, insbesondere Lenkrad, zum manuellen Greifen und Eingeben von Lenkbefehlen aufweist. Das Lenkrad ist an einem beweglichen und zusammenklappbaren Lenkradstützarm angelenkt und kann damit zwischen einer ausgefahrenen Gebrauchsposition, in der das Eingeben von Lenkbefehlen möglich ist, und einer eingefahrenen Nichtgebrauchsposition verlagert werden. In der Nichtgebrauchsposition ist das Lenkrad zusammen mit dem Lenkradstützarm in einem in einer Armlehne angeordneten Aufnahmeraum aufgenommen.

Die DE 11 2015 002 234 T5 beschreibt ein klimatisiertes Lenkrad mit einem Lenkradkranz, wobei zumindest der Lenkradkranz beheizt oder gekühlt werden kann.

Die vorliegende Erfindung beschäftigt sich mit dem Problem, für ein Fahrzeug der vorstehend beschriebenen Art eine verbesserte oder zumindest eine andere Ausführungsform anzugeben, die sich insbesondere durch eine effiziente Reinigung der Greiffläche auszeichnet. Ferner ist eine reduzierte Komplexität angestrebt.

Dieses Problem wird erfindungsgemäß durch den Gegenstand des unabhängigen Anspruchs gelöst. Vorteilhafte Ausführungsformen sind Gegenstand der abhängigen Ansprüche.

Die Erfindung beruht auf dem allgemeinen Gedanken, das Lenkeingabegerät verstellbar auszugestalten, derart, dass es zwischen einer ausgefahrenen Gebrauchsstellung, in der es für den Fahrzeugführer greifbar ist, und einer eingefahrenen Nichtgebrauchsstellung am Fahrzeug verstellbar angeordnet ist, in der es für den Fahrzeugführer nicht greifbar ist. Ferner wird vorgeschlagen, die Reinigungseinrichtung mit zumindest einer am Fahrzeug angeordneten Reinigungsbox auszustatten, die wenigstens eine Reinigungseinrichtung zum Reinigen der Greiffläche aufweist oder enthält. Des Weiteren sind die Verstellbarkeit des Lenkeingabegeräts und die Reinigungsbox so aufeinander abgestimmt, dass das Lenkeingabegerät in der Gebrauchsstellung zumindest im Bereich der Greiffläche außerhalb der Reinigungsbox angeordnet ist, während es in der Nichtgebrauchsstellung zumindest im Bereich der Greiffläche innerhalb der Reinigungsbox angeordnet ist. Durch die Unterbringung bzw. Positionierung des Lenkeingabegeräts in der Nichtgebrauchsstellung innerhalb der Reinigungsbox ergeben sich vielfältige vorteilhafte Möglichkeiten für die Ausgestaltung der jeweiligen Reinigungseinrichtung. Da die gesamte Greiffläche innerhalb der Reinigungsbox angeordnet ist, lassen sich beispielsweise flüssige Reinigungsmittel einsetzen, ohne dass die Gefahr besteht, dass dabei Reinigungsmittel in den Fahrzeuginnenraum gelangen und dort für eine Verunreinigung sorgt. Ferner nutzt die Erfindung die Erkenntnis, dass bei Fahrzeugen, die für ein autonomes Fahren ausgestaltet sind, häufig bereits ein Lenkeingabegerät vorgesehen ist, das sich von einer innerhalb des Fahrzeuginnenraums angeordneten Gebrauchsstellung in eine außerhalb des Fahrzeuginnenraums angeordnete Nichtgebrauchsstellung verstellen lässt. Beispielsweise kann das Lenkeingabegerät vollständig im Armaturenbrett aufgenommen werden. Jedenfalls ist auch bei derartigen Fahrzeugen angedacht, dass das Lenkeingabegerät in der jeweiligen Nichtgebrauchsstellung vom Fahrzeugführer nicht mehr greifbar ist. Wenn nun bei solchen Fahrzeugen ohnehin ein Aufnahmeraum für das Lenkeingabegerät für eine derartige Nichtgebrauchsstellung vorgesehen ist, kann zur Realisierung der hier vorgestellten Erfindung dieser ohnehin vorhandene Aufnahmeraum als Reinigungsbox konfiguriert bzw. genutzt werden. Somit lassen sich hier Synergien nutzen, wodurch sich die Komplexität des hier vorgestellten Fahrzeugs mit Reinigungsvorrichtung reduziert.

Die Greiffläche kann dabei insbesondere auch Taster, Schalter, Wippen und dergleichen umfassen.

Das Fahrzeug kann grundsätzlich jedes beliebige Fahrzeug sein, also insbesondere ein Landfahrzeug, ein Wasserfahrzeug oder ein Luftfahrzeug. Bevorzugt handelt es sich um ein Straßenfahrzeug und insbesondere um einen Personenkraftwagen.

Zweckmäßig steht also das Lenkeingabegerät in der Gebrauchsstellung in einen Fahrzeuginnenraum vor. Bei einer vorteilhaften Ausführungsform kann nun die Reinigungsbox zwischen einem Offenzustand, in dem das Lenkeingabegerät zwischen der Gebrauchsstellung und der Nichtgebrauchsstellung verstellbar ist, und einem Schließzustand verstellbar sein, in dem das in die Nichtgebrauchsstellung verstellte Lenkeingabegerät vom Fahrzeuginnenraum getrennt ist. Durch diese Trennung eines Boxinnenraums vom Fahrzeuginnenraum kann die Reinigung der Greiffläche am Lenkeingabegerät innerhalb des Boxinnenraums erfolgen, ohne dass es zu nachteiligen Wechselwirkungen mit dem Fahrzeuginnenraum kommt. Insbesondere lassen sich demnach flüssige und gasförmige Reinigungsmittel verwenden sowie Bestrahlungen durchführen.

Das Verstellen des Lenkeingabegeräts kann dabei grundsätzlich auf beliebige, geeignete Weise erfolgen. Denkbar ist beispielsweise ein lineares Verfahren des Lenkeingabegeräts. Ebenso ist ein einfaches oder mehrfaches Verschwenken oder Einklappen des Lenkeingabegeräts denkbar.

Um die Reinigungsbox zwischen dem Offenzustand und dem Schließzustand verstellen zu können, ist denkbar, die Reinigungsbox zweiteilig auszugestalten, sodass sie beispielsweise ein Boxunterteil und ein Boxoberteil aufweist, die relativ zueinander verstellbar sind. Ebenso ist denkbar, die Reinigungsbox mit einer Öffnung zum Fahrzeuginnenraum hin auszustatten, durch welche das Lenkeingabegerät in die Reinigungsbox einfahrbar ist bzw. daraus ausfahrbar ist. Die Reinigungsbox kann dann zusätzlich mit einem Deckel zum Verschließen dieser Öffnung ausgestattet sein. Insbesondere kann sich dieser Deckel im Schließzustand bündig in das Armaturenbrett einfügen.

Die Reinigungsbox kann mit einer einzigen Reinigungseinrichtung oder mit mehreren Reinigungseinrichtungen ausgestattet sein. Nachfolgend werden mehrere unterschiedliche Ausführungsformen für derartige Reinigungseinrichtungen vorgestellt, die alternativ und in beliebiger Kombination kumuliert zum Einsatz kommen können.

Beispielsweise kann eine solche Reinigungseinrichtung zum mechanischen Reinigen der Greiffläche ausgestaltet sein. Hierzu kann die Reinigungseinrichtung elastische Reinigungselemente aufweisen, wie z. B. Borsten, Finger, Noppen oder einen Schwamm. Ebenso kann ein mechanisches Reinigen mit einem Fluidstrahl nass oder trocken, also mit einem Flüssigkeitsstrahl oder mit einem Gasstrahl realisiert werden, wozu das jeweilige Fluid beispielsweise mit einem erhöhten Druck auf die Greiffläche aufgebracht wird. Hierdurch lassen sich insbesondere Essensreste und dergleichen besonders effizient von der Greiffläche entfernen.

Zweckmäßig kann vorgesehen sein, dass dieselbe oder eine andere Reinigungsvorrichtung zum Erzeugen einer Relativbewegung zwischen der jeweiligen Reinigungseinrichtung und der Greiffläche ausgestaltet ist. Dies ist insbesondere in Verbindung mit einer Reinigungseinrichtung zum mechanischen Reinigen der Greiffläche von Vorteil. Beispielsweise kann die Reinigungsbox relativ zum ortsfesten Lenkeingabegerät bewegt werden. Denkbar sind Schwingbewegungen, Vibrationen oder auch bidirektionale lineare oder drehende Bewegungen. Zusätzlich oder alternativ kann selbstverständlich auch das Lenkeingabegerät zum Erzeugen derartiger Relativbewegungen angesteuert werden. Ferner ist denkbar, die Reinigungselemente der mechanisch reinigenden Reinigungseinrichtung mit einem Gasstrom zu beaufschlagen, um die Reinigungselemente, wie z. B. Borsten, Finger, Noppen, relativ zur Greiffläche zu bewegen. Auch ist denkbar, dass eine Düse, die einen Fluidstrahl zum mechanischen Reinigen erzeugt, entlang der Greiffläche verstellt wird.

Bei einer anderen Ausführungsform kann zumindest eine solche Reinigungseinrichtung zum Beaufschlagen der Greiffläche mit einer Reinigungsflüssigkeit ausgestaltet sein, um mit Hilfe der Reinigungsflüssigkeit eine chemische Reinigung der Greiffläche zu bewirken. Die Reinigungsflüssigkeit kann insbesondere fettlösende Komponenten und/oder desinfizierende Komponenten beinhalten. Ferner kann vorgesehen sein, dass zumindest eine solche Reinigungseinrichtung erste Reinigungselemente zum mechanischen Reinigen der Greiffläche und zweite Reinigungselemente zum Beaufschlagen der ersten Reinigungselemente mit einer Reinigungsflüssigkeit aufweist. Bei dieser Ausführungsform wird nicht die Greiffläche direkt mit einer Reinigungsflüssigkeit beaufschlagt, sondern indirekt. Hierzu werden die ersten Reinigungselemente, die zum mechanischen Reinigen der Greiffläche dienen, also beispielsweise ein Schwamm, Borsten, Finger oder Noppen, mit der Reinigungsflüssigkeit beaufschlagt, sodass die mechanische Reinigung letztlich nass erfolgt und eine chemische Reinigung mit umfasst. Gleichzeitig werden hierdurch auch die ersten Reinigungselemente mitgereinigt.

Bei einer anderen Ausführungsform kann zumindest eine solche Reinigungseinrichtung zum Beaufschlagen der Greiffläche mit ultraviolettem Licht ausgestaltet sein.

Insbesondere ist dabei eine Beaufschlagung mit UVA- und/oder UVB- und/oder UVC-Licht denkbar.

Besonders vorteilhaft kann in diesem Zusammenhang eine Ausführungsform sein, bei welcher die Grifffläche mit Photokatalysatoren, wie z.B. Titanoxiden, insbesondere Titandioxid (TiO₂), ausgestattet ist, beispielsweise in Form einer Beschichtung der Greiffläche. Das UV-Licht kann nun eine zum Erzeugen einer photokatalytischen Selbstreinigung auf der Greiffläche geeignete Wellenlänge aufweisen. Durch diese Maßnahme lässt sich eine effiziente mikrobakterielle Reinigung der Grifffläche realisieren. In Verbindung mit Titanoxiden kann eine geeignete Wellenlänge im Bereich von 390 nm liegen.

Eine andere vorteilhafte Ausführungsform schlägt vor, dass zumindest eine solche Reinigungseinrichtung zum Beaufschlagen der Greiffläche mit einem flüssigen oder gasförmigen Desinfektionsmittel ausgestaltet ist. Durch die Desinfektion lassen sich insbesondere Bakterien, Pilze und auch Viren auf der Greiffläche abtöten. Bevorzugt kommt eine derartige Reinigungseinrichtung zum Desinfizieren in Verbindung mit einer Reinigungseinrichtung zum mechanischen und/oder chemischen Reinigen zum Einsatz, jedoch erst zeitlich nach der mechanischen bzw. chemischen Reinigung.

Eine andere vorteilhafte Ausführungsform schlägt vor, dass zumindest eine solche Reinigungseinrichtung zum Trocknen der Greiffläche ausgestaltet ist, so dass sie auch als Trocknungseinrichtung bezeichnet werden kann. Beispielsweise kann eine solche Reinigungseinrichtung als Heizung ausgestaltet sein, um die Lenkeinrichtung im Bereich der Greiffläche direkt zu erwärmen. Dies kann beispielsweise mittels Infrarotstrahlen erfolgen. Ebenso ist denkbar, dass die Reinigungseinrichtung zum Trocknen der Greiffläche einen temperierten Luftstrom erzeugt und damit die Greiffläche beaufschlagt.

Bei einer anderen Ausführungsform kann die Reinigungsvorrichtung wenigstens eine Pflegeeinrichtung zum Auftragen eines Pflegeprodukts auf die Greiffläche aufweisen. Die Greiffläche kann je nach Wertigkeit des Fahrzeugs aus Holz, Leder, Kunstleder oder allgemein aus Kunststoff bestehen. Die Reinigung kann die Greiffläche dementsprechend mehr oder weniger beanspruchen. Durch die Verwendung eines Pflegeprodukts, das beispielsweise eine Flüssigkeit, eine Paste oder eine Creme sein kann, lässt sich die Qualität der Greiffläche dauerhaft erhalten. Die Pflegeeinrichtung kann mit entsprechenden Pflegeelementen zum Applizieren des Pflegeprodukts ausgestattet sein. Beispielsweise sind auch hier wieder elastische Pflegeelemente denkbar, die unmittelbar mit der Greiffläche in Kontakt kommen können, wie z. B. Borsten, Finger, Noppen oder ein Schwamm. Auch hier ist denkbar, das Applizieren des Pflegeprodukts in Verbindung mit einer Relativbewegung zwischen dem Lenkeingabegerät und der Reinigungsbox oder zumindest der jeweiligen Pflegeeinrichtung bzw. des jeweiligen Pflegeelements zu unterstützen.

Eine andere vorteilhafte Ausführungsform sieht vor, dass die Reinigungsbox zum Temperieren des darin angeordneten Bereichs des Lenkeingabegeräts ausgestaltet ist. Beispielsweise kann das Lenkeingabegerät zumindest im Bereich der Greiffläche auf die im Fahrzeuginnenraum herrschende Temperatur angepasst werden. Denkbar ist hierzu ein Kühlen und/oder ein Erwärmen der Greiffläche. Dies kann bei stehendem Fahrzeug und auch während eines autonomen Fahrbetriebs des Fahrzeugs erfolgen. Für eine derartige Temperierung des Lenkeingabegeräts im Boxinnenraum kann die Reinigungsbox beispielsweise an ein Kanalsystem einer Klimatisierungsanlage, insbesondere an ein HVAC-System, angeschlossen sein, wobei HVAC für Heating, Ventilation and Air Conditioning steht. Somit kann ein temperierter Luftstrom dieser Fahrzeugklimatisierungsanlage bzw. des HVAC-Systems durch die Reinigungsbox geführt werden, wodurch das Lenkeingabegerät zumindest im Bereich der Grifffläche mit dem temperierten Luftstrom beaufschlagt wird.

Die Reinigungsbox kann in ihrem Innenraum im Wesentlichen wie eine Negativform des Lenkeingabegeräts zumindest im Bereich der Grifffläche geformt sein. Insbesondere können die für eine mechanische Reinigung der Grifffläche vorgesehenen Griffelemente entlang dieser Negativform verteilt angeordnet sein.

Die Reinigungseinrichtung kann außerdem mit einer Sensorik ausgestattet sein, die eine Verschmutzung der Grifffläche erkennt. Dabei kann es sich um Sensoren handeln, die unmittelbar in der Grifffläche angeordnet sind. Ebenso sind optische Sensoren denkbar, die an geeigneter Stelle des Fahrzeugs angeordnet sind und die Grifffläche abtasten. Des Weiteren kann die Reinigungseinrichtung mit einer Steuerung ausgestattet sein, die einen Reinigungsvorgang automatisch durchführen kann. Die Steuerung kann dabei mit einer Fahrzeugsteuerung gekoppelt sein, um beispielsweise einen autonomen Fahrbetrieb oder einen anderen Fahrzeugzustand zu identifizieren, der ein Reinigen der Greiffläche ermöglicht.

Weitere wichtige Merkmale und Vorteile der Erfindung ergeben sich aus den Unteransprüchen, aus den Zeichnungen und aus der zugehörigen Figurenbeschreibung anhand der Zeichnungen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind. Vorstehend genannte und nachfolgend noch zu nennende Bestandteile einer übergeordneten Einheit, wie z.B. einer Einrichtung, einer Vorrichtung oder einer Anordnung, die separat bezeichnet sind, können separate Bauteile bzw. Komponenten dieser Einheit bilden oder integrale Bereiche bzw. Abschnitte dieser Einheit sein, auch wenn dies in den Zeichnungen anders dargestellt ist.

Bevorzugte Ausführungsbeispiele der Erfindung sind in den Zeichnungen dargestellt und werden in der nachfolgenden Beschreibung näher erläutert, wobei sich gleiche Bezugszeichen auf gleiche oder ähnliche oder funktional gleiche Komponenten beziehen.

Es zeigen, jeweils schematisch,
- Fig. 1: eine stark vereinfachte, schaltplanartige Prinzipdarstellung eines Fahrzeugs mit Lenkeinrichtung und Reinigungsvorrichtung, wobei ein Lenkeingabegerät in eine Gebrauchsstellung verstellt ist,
- Fig. 2: eine Ansicht wie in Figur 1, wobei das Lenkeingabegerät in eine Nichtgebrauchsstellung verstellt ist,
- Fig. 3 bis 7: jeweils eine stark vereinfachte Schnittansicht durch eine Reinigungsbox mit unterschiedlichen Reinigungseinrichtungen,
- Fig. 8 bis 10: vereinfachte isometrische Ansichten des Fahrzeugs im Bereich des Lenkeingabegeräts in unterschiedlichen Stellungen,
- Fig. 11 bis 13: Ansichten wie in den Figuren 8 bis 10, jedoch bei einem anderen Lenkeingabegerät.

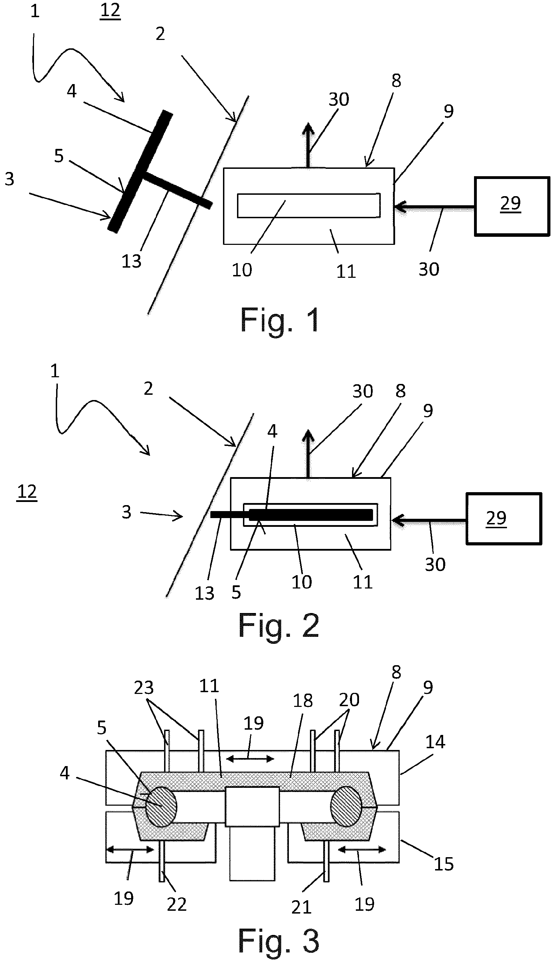

Entsprechend den Figuren 1 und 2 umfasst ein Fahrzeug 1, das in einem vereinfachten Schnitt quer zu einem Armaturenbrett 2 wiedergegeben ist, eine Lenkeinrichtung 3 zum Lenken des Fahrzeugs 1, die ein manuell betätigbares Lenkeingabegerät 4 aufweist, mit dem der Fahrzeugführer manuell Lenkbefehle in die Lenkeinrichtung 3 eingeben kann.

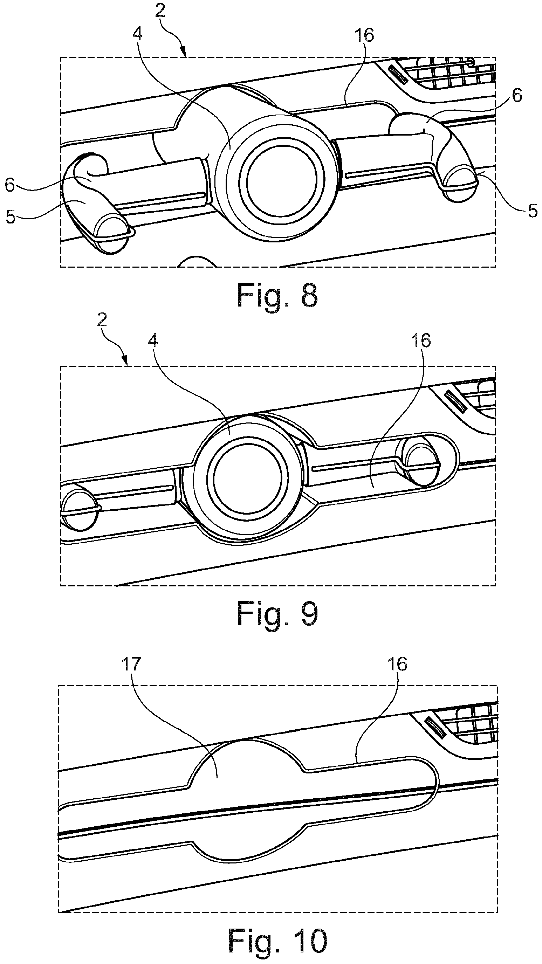

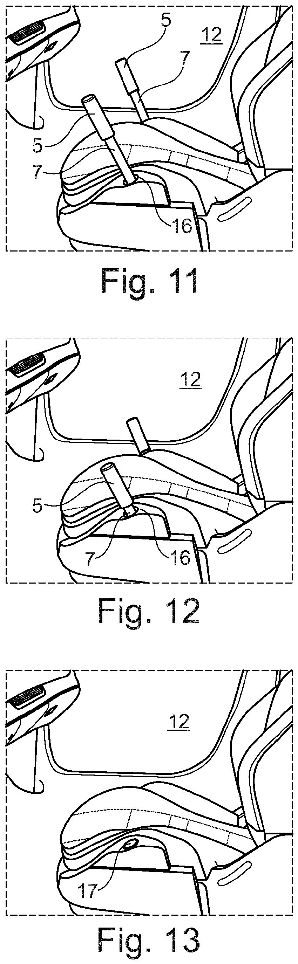

Hierzu kann der Fahrzeugführer das Lenkeingabegerät 4 manuell greifen. Hierzu weist das Lenkeingabegerät 4 eine einteilige oder mehrteilige Greiffläche 5 auf, die vom Fahrzeugführer zum Eingeben von Lenkbefehlen in die Lenkeinrichtung 3 manuell greifbar ist. Im Beispiel der Figuren 1 und 2 ist das Lenkeingabegerät 4 als Lenkrad ausgestaltet, das eine einteilige, entlang des Umfangs umlaufende Greiffläche 5 aufweist. Rein exemplarisch ist in den Figuren 8 und 9 ein anderes Lenkeingabegerät 4 wiedergegeben, das zwei Griffelemente 6 aufweist, im Übrigen jedoch wie ein Lenkrad betätigbar ist. In diesem Fall ist die Greiffläche 5 mehrteilig und auf die beiden Griffelemente 6 verteilt. Weiter zeigen die Figuren 11 bis 13 rein exemplarisch ein Lenkeingabegerät 4, das mit zwei Lenkstangen 7 gebildet ist. Auch hier ist die Greiffläche 5 mehrteilig und auf die beiden Lenkstangen 7 verteilt.

Gemäß den Figuren 1 und 2 ist das Fahrzeug 1 außerdem mit einer Reinigungsvorrichtung 8 zum Reinigen der Greiffläche 5 ausgestattet. Die Reinigungsvorrichtung 8 weist eine am Fahrzeug 1 bzw. im Armaturenbrett 2 angeordnete Reinigungsbox 9 auf, die einen Boxinnenraum 10 umschließt und die wenigstens eine mit Bezug auf die Figuren 3 bis 7 näher erläuterte Reinigungseinrichtung 11 aufweist bzw. enthält. Im Beispiel der Fig. 11 bis 13 weist die Reinigungsvorrichtung 8 zwei Reinigungsboxen auf, die neben einem Fahrzeugsitz in einer Bodenstruktur eines Fahrzeuginnenraums 12 angeordnet sind.

Das Lenkeingabegerät 4 ist zwischen einer in Figur 1 wiedergegebenen Gebrauchsstellung, in der es für den Fahrzeugführer greifbar ist, und einer in Figur 2 wiedergegebenen Nichtgebrauchsstellung verstellbar, in der es für den Fahrzeugführer nicht greifbar ist. In der Gebrauchsstellung befindet sich das Lenkeingabegerät 4 gemäß Figur 1 außerhalb der Reinigungsbox 9, wobei es in den Fahrzeuginnenraum 12 vorsteht. Gemäß Figur 2 befindet sich das Lenkeingabegerät 4 in der Nichtgebrauchsstellung in der Reinigungsbox 9. Hierzu kann ein Lenkgestänge 13, welches das Lenkeingabegerät 4 mit der übrigen Lenkeinrichtung 3 verbindet, faltbar oder klappbar oder teleskopierbar oder in sonstiger geeigneter Weise verstellbar ausgebildet sein.

Die Reinigungsbox 9 ist in ihrem Boxinnenraum 10 zweckmäßig als Negativform zum jeweiligen Lenkeingabegerät 4 konzipiert. Dies kann beispielsweise mittels der jeweils verwendeten Reinigungseinrichtung 11 erfolgen. Ferner kann zweckmäßig vorgesehen sein, dass die Reinigungsbox 9 zwischen einem Offenzustand und einem Schließzustand verstellbar ist. In den Figuren 1 bis 7, 10 und 13 ist jeweils ein Schließzustand gezeigt, während die Figuren 8, 9, 11 und 12 einen Offenzustand zeigen. Im Offenzustand ist das Lenkeingabegerät 4 zwischen der Gebrauchsstellung und der Nichtgebrauchsstellung verstellbar. Im Schließzustand ist das Lenkeingabegerät 4, wenn es in die Nichtgebrauchsstellung verstellt ist, vom Fahrzeuginnenraum 12 getrennt. In den Beispielen der Figuren 3 bis 7 ist hierzu die Reinigungsbox 9 zweiteilig konzipiert, derart, dass sie ein Oberteil 14 und ein Unterteil 15 aufweist, die relativ zueinander verstellbar sind, um den Offenzustand und den Schließzustand zu ermöglichen. In den Beispielen der Figuren 8 bis 10 und 11 bis 13 ist dagegen vorgesehen, dass die Reinigungsbox 9 eine Öffnung 16 aufweist, durch die hindurch das Lenkeingabegerät 4 zwischen dem Fahrzeuginnenraum 12 und dem Boxinnenraum 10 verstellbar ist. Die Reinigungsbox 9 ist dann zweckmäßig außerdem mit einem Deckel 17 zum Verschließen dieser Öffnung 16 ausgestattet, um den Schließzustand zu erzeugen. Im Beispiel der Figur 10 ist der Deckel 17 im Schließzustand im Armaturenbrett 2 bündig angeordnet. Im Beispiel der Figuren 11 bis 13 wird der Deckel 17 durch ein stirnseitiges Ende der jeweiligen Lenkstange 7 gebildet.

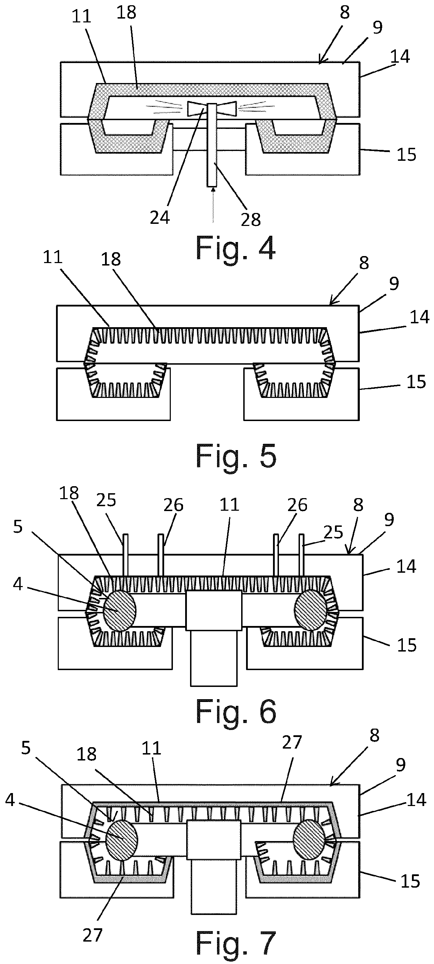

Zumindest eine Reinigungseinrichtung 11 kann zum mechanischen Reinigen der Grifffläche 5 ausgestaltet sein. Gemäß Figur 3 kann die Reinigungseinrichtung 11 hierzu beispielsweise ein elastisches Reinigungselement 18 aufweisen, das unmittelbar mit der Greiffläche 5 in Kontakt kommt, um diese mechanisch zu reinigen. Gemäß den Figuren 3 und 4 kann es sich bei diesem Reinigungselement 18 beispielsweise um einen Schwamm handeln. Gemäß den Figuren 5 bis 7 kann es sich bei diesem Reinigungselement 18 um eine Vielzahl von Borsten oder Finger oder Noppen handeln.

Gemäß Figur 3 kann die Reinigungsvorrichtung 8 zum Erzeugen einer durch Doppelpfeile angedeuteten Relativbewegung 19 zwischen der jeweiligen Reinigungseinrichtung 11 bzw. dem jeweiligen Reinigungselement 18 und der Greiffläche 5 ausgestaltet sein. Beispielsweise kann die gesamte Reinigungsbox 9 bewegt werden, um die Relativbewegung 19 zu erzeugen. Zusätzlich oder alternativ ist auch denkbar, das Lenkeingabegerät 4 entsprechend zu bewegen.

Im Beispiel der Figur 3 kann die Reinigungseinrichtung 11 zusätzlich so ausgestaltet sein, dass damit die Greiffläche 5 mit einer Reinigungsflüssigkeit beaufschlagt werden kann. In Figur 3 sind entsprechende Zulaufleitungen zum Zuführen der Reinigungsflüssigkeit sowie eine Ablaufleitung 21 zum Abführen der Reinigungsflüssigkeit wiedergegeben. Zusätzlich kann die Reinigungseinrichtung 11 auch dazu konzipiert sein, die Greiffläche 5 mit einem flüssigen oder gasförmigen Desinfektionsmittel zu beaufschlagen. Dieses lässt sich beispielsweise ebenfalls durch die Zuführleitungen 20 zuführen und ggf. durch die Abführleitung 21 abführen. Des Weiteren kann diese oder eine andere Reinigungseinrichtung 11 zum Trocknen der Greiffläche 5 ausgestaltet sein. Beispielsweise lässt sich über eine weitere Zuführleitung 22 ein getrockneter und/oder beheizter Luftstrom dem Boxinnenraum 10 zuführen, um die Greiffläche 5 zu trocknen. Die Abluft kann über entsprechende Abführleitungen 23 abgeführt werden.

Gemäß Figur 4 ist auch denkbar, die jeweilige Reinigungseinrichtung 11 so zu konzipieren, dass die zum mechanischen Reinigen vorgesehenen ersten Reinigungselemente 18 mit Hilfe zweiter Reinigungselemente 24 mit einer Reinigungsflüssigkeit beaufschlagt werden, was zweckmäßig vor dem Überführen des Lenkeingabegeräts 4 in die Reinigungsbox 9 erfolgt. Der mechanische Reinigungsvorgang kann dann nass, also in Verbindung mit der Reinigungsflüssigkeit durchgeführt werden.

Gemäß den Figuren 5 bis 7 können zur mechanischen Reinigung der Greiffläche 5 fingerartige oder noppenartige Reinigungselemente 18 zum Einsatz kommen. Eine Relativbewegung dieser Reinigungselemente 18 gegenüber der Greiffläche 5 lässt sich gemäß Figur 6 auch dadurch realisieren, dass über Zuführleitungen 25 Druckluft, insbesondere impulsartig, zugeführt wird, wobei die Druckluft über Abführleitungen 26 wieder abgeführt werden kann.

Gemäß Figur 7 kann diese oder eine andere Reinigungseinrichtung 11 zum Beaufschlagen der Greiffläche 5 mit UV-Licht ausgestaltet sein. Beispielsweise kann an geeigneter Stelle an einer dem Boxinnenraum 10 zugewandten Innenseite der Reinigungsbox 9 eine entsprechende UV-Lichtquelle 27 angeordnet sein, die eine UV-Lichtbestrahlung der Greiffläche 5 ermöglicht. Insbesondere kann gemäß Figur 7 der gesamte Boxinnenraum 10 mit UV-Licht beaufschlagt werden, sodass gleichzeitig auch eine UV-Bestrahlung der Reinigungselemente 18 erfolgt. Mit Hilfe von UV-Licht lässt sich eine Vielzahl mikrobakterieller Verunreinigungen abtöten.

Optional kann die Greiffläche 5 mit Photokatalysatoren ausgestattet sein, beispielsweise in Form einer entsprechenden Beschichtung. Das UV-Licht kann nun zum Erzeugen einer photokatalytischen Selbstreinigung auf der Greiffläche 5 konzipiert sein und dementsprechend eine hierfür geeignete Wellenlänge aufweisen. Bei der Verwendung von Titanoxiden als Photokatalysatoren kann beispielsweise eine Wellenlänge von 390 nm geeignet sein.

Die Reinigungsvorrichtung 8 kann außerdem eine Pflegeeinrichtung 28 aufweisen, mit deren Hilfe sich ein Pflegeprodukt auf die Greiffläche 5 auftragen lässt. Gemäß Figur 4 kann hierzu grundsätzlich ebenfalls eine Düse oder dergleichen zum Einsatz kommen, die sich auch zum Zuführen einer Reinigungsflüssigkeit eignet. Bevorzugt werden hierbei separate Systeme verwendet. Beispielsweise ist denkbar, nach dem Reinigen und nach dem Desinfizieren der Greiffläche 5 sowie nach dem Trocknen der Greiffläche 5 das Pflegeprodukt aufzutragen. Im Anschluss daran kann nach dem Verstellen des Lenkeingabegeräts 4 in dessen Gebrauchsstellung eine Selbstreinigung der Reinigungselemente 18 durchgeführt werden, beispielsweise um diese wieder von den Pflegeprodukten zu befreien.

Gemäß den Figuren 1 und 2 kann die Reinigungsbox 9 zum Temperieren des im Boxinnenraum 10 angeordneten Lenkeingabegeräts 4 ausgestaltet sein. Beispielsweise kann hierzu die Reinigungsbox 9 an eine Klimatisierungsanlage 29 angeschlossen sein. Beispielsweise kann die Reinigungsbox 9 in ein Kanalsystem 30 der Klimatisierungsanlage 29 eingebunden sein, sodass der Boxinnenraum 9 von im Kanalsystem 30 strömender klimatisierter Luft durchströmt wird. Somit lässt sich das Lenkeingabegerät 4 wunschgemäß temperieren, also insbesondere kühlen oder erwärmen.

## Patentansprüche

1. Fahrzeug (1) mit einer Lenkeinrichtung (3) zum Lenken des Fahrzeugs (1), die ein manuell betätigbares Lenkeingabegerät (4) zum manuellen Greifen und Eingeben von Lenkbefehlen aufweist, wobei das Lenkeingabegerät (4) eine einteilige oder mehrteilige Greiffläche (5) aufweist, die vom Fahrzeugführer zum Eingeben von Lenkbefehlen manuell greifbar ist, und
mit einer Reinigungsvorrichtung (8) zum Reinigen der Greiffläche (5),
**dadurch gekennzeichnet,**
- **dass** das Lenkeingabegerät (4) zwischen einer Gebrauchsstellung, in der es für den Fahrzeugführer greifbar ist, und einer Nichtgebrauchsstellung verstellbar am Fahrzeug (1) angeordnet ist, in der es für den Fahrzeugführer nicht greifbar ist,
- **dass** die Reinigungsvorrichtung (8) wenigstens eine am Fahrzeug (1) angeordnete Reinigungsbox (9) aufweist, die wenigstens eine Reinigungseinrichtung (11) zum Reinigen der Greiffläche (5) aufweist oder enthält, und
- **dass** das Lenkeingabegerät (4) in der Gebrauchsstellung zumindest im Bereich der Greiffläche (5) außerhalb der Reinigungsbox (9) angeordnet ist, während es in der Nichtgebrauchsstellung zumindest im Bereich der Greiffläche (5) innerhalb der Reinigungsbox (9) angeordnet ist.

2. Fahrzeug (1) nach Anspruch 1,
**dadurch gekennzeichnet,**
- **dass** das Lenkeingabegerät (4) in der Gebrauchsstellung in einen Fahrzeuginnenraum (12) vorsteht,
- **dass** die Reinigungsbox (9) zwischen einem Offenzustand, in dem das Lenkeingabegerät (4) zwischen der Gebrauchsstellung und der Nichtgebrauchsstellung verstellbar ist, und einem Schließzustand verstellbar ist, in dem das in die Nichtgebrauchsstellung verstellte Lenkeingabegerät (4) vom Fahrzeuginnenraum (12) getrennt ist.

3. Fahrzeug (1) nach Anspruch 1 oder 2,
**dadurch gekennzeichnet,**
- **dass** zumindest eine Reinigungseinrichtung (11) oder die Reinigungseinrichtung (11) zum mechanischen Reinigen der Greiffläche (5) ausgestaltet ist.

4. Fahrzeug (1) nach einem der Ansprüche 1 bis 3,
**dadurch gekennzeichnet,**
- **dass** die Reinigungsvorrichtung (8) zum Erzeugen einer Relativbewegung (19) zwischen der jeweiligen Reinigungseinrichtung (11) und der Greiffläche (5) ausgestaltet ist.

5. Fahrzeug (1) nach einem der Ansprüche 1 bis 4,
**dadurch gekennzeichnet,**
- **dass** zumindest eine Reinigungseinrichtung (11) oder die Reinigungseinrichtung (11) zum Beaufschlagen der Greiffläche (5) mit einer Reinigungsflüssigkeit ausgestaltet ist, oder
- **dass** zumindest eine Reinigungseinrichtung (11) oder die Reinigungseinrichtung (11) erste Reinigungselemente (18) zum mechanischen Reinigen der Greiffläche (5) und zweite Reinigungselemente (24) zum Beaufschlagen der ersten Reinigungselemente (18) mit einer Reinigungsflüssigkeit aufweist.

6. Fahrzeug (1) nach einem der Ansprüche 1 bis 5,
**dadurch gekennzeichnet,**
- **dass** zumindest eine Reinigungseinrichtung (11) oder die Reinigungseinrichtung (11) zum Beaufschlagen der Greiffläche (5) mit UV-Licht ausgestaltet ist.

7. Fahrzeug (1) nach einem der Ansprüche 1 bis 6,
**dadurch gekennzeichnet,**
- **dass** zumindest eine Reinigungseinrichtung (11) oder die Reinigungseinrichtung (11) zum Beaufschlagen der Greiffläche (5) mit einem flüssigen oder gasförmigen Desinfektionsmittel ausgestaltet ist.

8. Fahrzeug (1) nach einem der Ansprüche 1 bis 7,
**dadurch gekennzeichnet,**
- **dass** zumindest eine Reinigungseinrichtung (11) oder die Reinigungseinrichtung (11) zum Trocknen der Greiffläche (5) ausgestaltet ist.

9. Fahrzeug (1) nach einem der Ansprüche 1 bis 8,
**dadurch gekennzeichnet,**
- **dass** die Reinigungsvorrichtung (8) wenigstens eine Pflegeeinrichtung (28) zum Auftragen eines Pflegeprodukts auf die Greiffläche (5) aufweist.

10. Fahrzeug (1) nach einem der Ansprüche 1 bis 9,
**dadurch gekennzeichnet,**
- **dass** die Reinigungsbox (9) zum Temperieren des darin angeordneten Bereichs des Lenkeingabegeräts (4) ausgestaltet ist.

## Claims

1. Vehicle (1) having a steering device (3) for steering the vehicle (1), which steering device has a manually operable steering input unit (4) for manually gripping and inputting steering commands, wherein the steering input unit (4) has a one-piece or multi-piece gripping surface (5) which can be manually gripped by the vehicle driver for inputting steering commands, and
having a cleaning apparatus (8) for cleaning the gripping surface (5),
**characterized in that**
- the steering input unit (4) is arranged on the vehicle (1) so as to be adjustable between a usage position, in which it can be gripped by the vehicle driver, and a non-usage position, in which it cannot be gripped by the vehicle driver,
- the cleaning apparatus (8) has at least one cleaning box (9) arranged on the vehicle (1), which cleaning box has or contains at least one cleaning device (11) for cleaning the gripping surface (5), and
- the steering input unit (4), in the usage position, is arranged outside the cleaning box (9), at least in the region of the gripping surface (5), while, in the non-usage position, it is arranged inside the cleaning box (9), at least in the region of the gripping surface (5).

2. Vehicle (1) according to claim 1,
**characterized in that**
- the steering input unit (4) projects into a vehicle interior (12) in the usage position,
- the cleaning box (9) is adjustable between an open state, in which the steering input unit (4) is adjustable between the usage position and the non-usage position, and a closed state, in which the steering input unit (4) adjusted into the non-usage position is separated from the vehicle interior (12).

3. Vehicle (1) according to claim 1 or 2,
**characterized in that**
- at least one cleaning device (11) or the cleaning device (11) is designed for mechanically cleaning the gripping surface (5).

4. Vehicle (1) according to any of claims 1 to 3,
**characterized in that**
- the cleaning apparatus (8) is designed to generate a relative movement (19) between the particular cleaning device (11) and the gripping surface (5).

5. Vehicle (1) according to any of claims 1 to 4,
**characterized in that**
- at least one cleaning device (11) or the cleaning device (11) is designed to apply a cleaning liquid to the gripping surface (5), or
- at least one cleaning device (11) or the cleaning device (11) has first cleaning elements (18) for mechanically cleaning the gripping surface (5) and second cleaning elements (24) for applying a cleaning liquid to the first cleaning elements (18).

6. Vehicle (1) according to any of claims 1 to 5,
**characterized in that**
- at least one cleaning device (11) or the cleaning device (11) is designed to apply UV light to the gripping surface (5).

7. Vehicle (1) according to any of claims 1 to 6,
**characterized in that**
- at least one cleaning device (11) or the cleaning device (11) is designed to apply a liquid or gaseous disinfectant to the gripping surface (5).

8. Vehicle (1) according to any of claims 1 to 7,
**characterized in that**
- at least one cleaning device (11) or the cleaning device (11) is designed to dry the gripping surface (5).

9. Vehicle (1) according to any of claims 1 to 8,
**characterized in that**
- the cleaning apparatus (8) has at least one care device (28) for applying a care product to the gripping surface (5).

10. Vehicle (1) according to any of claims 1 to 9,
**characterized in that**
- the cleaning box (9) is designed to control the temperature of the region of the steering input unit (4) arranged therein.

## Revendications

1. Véhicule (1) comportant un dispositif de direction (3) pour la direction du véhicule (1), qui présente un appareil d'entrée de direction (4) actionnable manuellement pour la saisie et l'entrée manuelles d'instructions de direction, dans lequel l'appareil d'entrée de direction (4) présente une surface de préhension (5) en une ou plusieurs parties, qui peut être saisie manuellement par le conducteur de véhicule pour l'entrée d'instructions de direction, et
comportant un dispositif de nettoyage (8) pour le nettoyage de la surface de préhension (5),
**caractérisé en ce**
- **que** l'appareil d'entrée de direction (4) est disposé de manière réglable sur le véhicule (1) entre une position d'utilisation, dans laquelle il peut être saisi par le conducteur de véhicule, et une position de non-utilisation, dans laquelle il ne peut pas être saisi par le conducteur de véhicule,
- **que** le dispositif de nettoyage (8) présente au moins une boîte de nettoyage (9) disposée sur le véhicule (1), qui présente ou contient au moins un dispositif de nettoyage (11) pour le nettoyage de la surface de préhension (5), et
- **que** l'appareil d'entrée de direction (4) est disposé, dans la position d'utilisation, au moins dans la zone de la surface de préhension (5), à l'extérieur de la boîte de nettoyage (9), tandis que, dans la position de non-utilisation, il est disposé au moins dans la zone de la surface de préhension (5), à l'intérieur de la boîte de nettoyage (9).

2. Véhicule (1) selon la revendication 1,
**caractérisé en ce**
- **que** l'appareil d'entrée de direction (4) fait saillie dans un habitacle de véhicule (12) dans la position d'utilisation,
- **que** la boîte de nettoyage (9) peut être déplacée entre un état ouvert, dans lequel l'appareil d'entrée de direction (4) peut être déplacé entre la position d'utilisation et la position de non-utilisation, et un état fermé, dans lequel l'appareil d'entrée de direction (4) déplacé dans la position de non-utilisation est séparé de l'habitacle de véhicule (12).

3. Véhicule (1) selon la revendication 1 ou 2,
**caractérisé en ce**
- **qu'**au moins un dispositif de nettoyage (11) ou le dispositif de nettoyage (11) est conçu pour le nettoyage mécanique de la surface de préhension (5).

4. Véhicule (1) selon l'une des revendications 1 à 3,
**caractérisé en ce**
- **que** le dispositif de nettoyage (8) est conçu pour la génération d'un mouvement relatif (19) entre le dispositif de nettoyage (11) respectif et la surface de préhension (5).

5. Véhicule (1) selon l'une des revendications 1 à 4,
**caractérisé en ce**
- **qu'**au moins un dispositif de nettoyage (11) ou le dispositif de nettoyage (11) est conçu pour l'exposition de la surface de préhension (5) à un liquide de nettoyage, ou
- **qu'**au moins un dispositif de nettoyage (11) ou le dispositif de nettoyage (11) présente des premiers éléments de nettoyage (18) pour le nettoyage mécanique de la surface de préhension (5) et des seconds éléments de nettoyage (24) pour l'exposition des premiers éléments de nettoyage (18) à un liquide de nettoyage.

6. Véhicule (1) selon l'une des revendications 1 à 5,
**caractérisé en ce**
- **qu'**au moins un dispositif de nettoyage (11) ou le dispositif de nettoyage (11) est conçu pour l'exposition de la surface de préhension (5) à la lumière UV.

7. Véhicule (1) selon l'une des revendications 1 à 6,
**caractérisé en ce**
- **qu'**au moins un dispositif de nettoyage (11) ou le dispositif de nettoyage (11) est conçu pour l'exposition de la surface de préhension (5) à un agent désinfectant liquide ou gazeux.

8. Véhicule (1) selon l'une des revendications 1 à 7,
**caractérisé en ce**
- **qu'**au moins un dispositif de nettoyage (11) ou le dispositif de nettoyage (11) est conçu pour le séchage de la surface de préhension (5).

9. Véhicule (1) selon l'une des revendications 1 à 8,
**caractérisé en ce**
- **que** le dispositif de nettoyage (8) présente au moins un dispositif d'entretien (28) pour l'application d'un produit d'entretien sur la surface de préhension (5).

10. Véhicule (1) selon l'une des revendications 1 à 9,
**caractérisé en ce**
- **que** la boîte de nettoyage (9) est conçue pour le tempérage de la zone de l'appareil d'entrée de direction (4) qui y est disposé.
